# EUROPEAN PATENT SPECIFICATION

(11) **EP 4 482 540 B1**
(45) Date of publication and mention of the grant of the patent: **28.10.2026**
(21) Application number: 23704369.0
(22) Date of filing: 16.02.2023
(51) Int. Cl.: A61L 9/20, F21V 3/04, F21V 7/22, F21V 33/00, F21Y 113/10, F21Y 115/10

(54) **HYBRID UV-WHITE LIGHT SOURCE**
HYBRIDE UV-WEISS-LICHTQUELLE
SOURCE DE LUMIÈRE BLANCHE UV HYBRIDE

(30) Priority: 22.02.2022 EP 22157900
(43) Date of publication of application: 01.01.2025
(73) Proprietor: Signify Holding B.V., 5656 AE Eindhoven (NL)
(72) Inventor: VAN BOMMEL, Ties, 5656 AE Eindhoven (NL)
(74) Representative: Verweij, Petronella Daniëlle
(86) International application number: PCT/EP2023/053846
(87) International publication number: WO 2023/161108

(56) References cited:
- CN-B- 106 195 674
- KR-A- 20190 110 972
- US-A1- 2010 290 208
- US-A1- 2018 209 609

## Description

### FIELD OF THE INVENTION

The invention relates to a light generating system as well as to a lighting device comprising such light generating system. The invention also relates to a method for providing light in a space.

### BACKGROUND OF THE INVENTION

Lamps comprising different LEDs are known in the art. US2013/0154519, for instance, describes a white-light-emitting device comprising: a plurality of ultraviolet or blue light-emitting diodes (LEDs) having at least one emission surface; a conversion coating spaced away from but enveloping the at least one emission surface to define a first mixing cavity therearound, the conversion coating converting a color of at least a portion of the light emitted by the plurality of LEDs to a different color; at least one secondary LED, emitting light of a color different from ultraviolet and blue, spaced away from the first mixing cavity; and a diffuser spaced away from but enveloping the conversion coating and the secondary LED to define therearound a second mixing cavity that is unfilled, wherein mixing of the converted light and the light from the at least one secondary LED produces white light that is emitted through the diffuser.

US2018/209609A discloses a lighting assembly that comprises a first light source, a second light source and an UV filter. The first light source emits in operation visible light. The second light source emits in operation UV light. The UV filter allows a transmission of visible light and absorbs or reflects UV light. The UV filter is arranged in an optical path from the second light source towards the first light source to prevent the UV light to impinge on the first light source while allowing the emission of the UV light into the ambient of the lighting assembly.

CN106195674B discloses a light source with a combination of a black light source and a signaling source that indicates the black light emitting region.

US2010/290208A discloses a lighting device comprising a first group of solid state light emitters comprising at least a first solid state light emitter, a second group of solid state light emitters comprising at least a second solid state light emitter, and a luminescent material-containing element comprising at least one luminescent material. The second group of solid state light emitters is spaced from the luminescent material-containing element, wherein at least 50% of the light emitted by the first solid state light emitter does not mix with any light emitted by any of the second group of solid state light emitters until after the light emitted by the second solid state light emitters has entered the luminescent material-containing element.

KR20190110972A discloses a LED module including at least one light emitting diode (LED), at least one sterilizing LED for removing bacteria, and a substrate on which the LED and the sterilizing LED are mounted.

### SUMMARY OF THE INVENTION

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore, the selection of wavelength of radiation, intensity of radiation and duration of irradiation may be limited in environments where people may reside such as offices, public transport, cinema's, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS, and MERS.

It appears desirable to produce systems, that provide alternative ways for air treatment, such as disinfection. Further, existing systems for disinfection may not easily be implemented in existing infrastructure, such as in existing buildings like offices, hospitality areas, etc. and/or may not easily be able to serve larger spaces. This may again increase the risk of contamination. Further, incorporation in HVAC systems may not lead to desirable effects and appears to be relatively complex. Further, existing systems may not be efficient, or may be relatively bulky, and may also not easily be incorporated in functional devices, such as e.g. luminaires.

Other disinfection systems may use one or more anti-microbial and/or antiviral means to disinfect a space or an object. Examples of such means may be chemical agents which may raise concerns. For instance, the chemical agents may also be harmful for people and pets.

In embodiments, the disinfecting light, may especially comprise ultraviolet (UV) radiation (and/or optionally violet radiation), i.e., the light may comprise a wavelength selected from the ultraviolet wavelength range (and/or optionally the violet wavelength range). However, other wavelengths are herein not excluded. The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV, violet, and NIR wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared (deep red) | NIR | 780-950 | + | +/- | +++ | | |
| Violet | V | 380-420 | +/- | - | + | | |
| Ultraviolet A | UV-A | 315-380 | + | - | + | | |
| Ultraviolet B | UV-B | 280-315 | + | +/- | +/- | + | |
| Near ultraviolet C | Near UV-C | 230-280 | ++ | ++ | - | | |
| Far ultraviolet C | Far UV-C | 190-230 | +++ | +++ | + | | +/- |
| Extreme ultraviolet C | Extreme UV-C | 100-190 | +++ | +++ | - | | + |

Each UV type / wavelength range may have different benefits and/or drawbacks. Relevant aspects may be (relative) sterilization effectiveness, safety (regarding radiation), and ozone production (as result of its radiation). Depending on an application a specific type of UV light or a specific combination of UV light types may be selected and provides superior performance over other types of UV light. UV-A may be (relatively) safe and may inactivate (kill) bacteria, but may be less effective in inactivating (killing) viruses. UV-B may be (relatively) safe when a low dose (i.e. low exposure time and/or low intensity) is used, may inactivate (kill) bacteria, and may be moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV-C may also be effective in inactivating (killing) bacteria and viruses, but may be (relatively to other UV-C wavelength ranges) (rather) safe. Far-UV light may generate some ozone which may be harmful for human beings and animals. Extreme UV-C may also be effective in inactivating (killing) bacteria and viruses, but may be relatively unsafe. Extreme UV-C may generate ozone which may be undesired when exposed to human beings or animals. In some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired. The types of light indicated in above table may in embodiments be used to sanitize air and/or surfaces.

The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, in embodiments, the light may comprise a wavelength in the UV-A range. In further embodiments, the light may comprise a wavelength in the UV-B range. In further embodiments, the light may comprise a wavelength in the Near UV-C range. In further embodiments, the light may comprise a wavelength in the Far UV-C range. In further embodiments, the light may comprise a wavelength in the extreme UV-C range. The Near UV-C, the Far UV-C and the extreme UV-C ranges may herein also collectively be referred to as the UV-C range. Hence, in embodiments, the light may comprise a wavelength in the UV-C range. In other embodiments, the light may comprise violet radiation.

Light or radiation described herein may also be indicated as disinfection light when the light comprises UV radiation.

UV LEDs are currently less efficient than white LEDs. Hence, a combination of such light sources may lead to system having less desirable properties, such as too much loss of UV radiation. Further, current lamps may not have a disinfection function and/or current disinfection devices may not have a lighting function. However, there appears to be a desire to combine such functions. Further, there appears to be a desire to efficiently use existing infrastructures.

Hence, it is an aspect of the invention to provide an alternative light generating system, which preferably further at least partly obviates one or more of above-described drawbacks. The present invention may have as object to overcome or ameliorate at least one of the disadvantages of the prior art, or to provide a useful alternative.

The invention provides a light generating system ("system") according to claim 1.

With such system(s) relatively efficient UV radiation and visible light may be produced. Further, homogeneity of the visible light such as color over angle effects and/or spottiness may be reduced. Further, such system may allow using existing infrastructures for providing visible light to also use for providing disinfection light. Here below, some general embodiments relating to light generating devices are described, which may individually apply to the first light generating device and the second light generating device. Especially, the light generating device may comprise a light source. The light source may especially configured to generate light source light. In embodiments, the device light may essentially consist of the device light. In other embodiments, the device light may essentially consist of converted light source light. In yet other embodiments, the device light may comprise (unconverted) light source light and converted light source light. Light source light may be converted with a luminescent material into luminescent material light and/or with an upconverter into upconverted light (see also below). The term "light generating device" may also refer to a plurality of light generating devices which may provide device light having essentially the same spectral power distributions. In specific embodiments, the term "light generating device" may also refer to a plurality of light generating devices which may provide device light having different spectral power distributions.

The term "light source" may in principle relate to any light source known in the art. It may be a conventional (tungsten) light bulb, a low pressure mercury lamp, a high pressure mercury lamp, a fluorescent lamp, a LED (light emissive diode). In a specific embodiment, the light source comprises a solid state LED light source (such as a LED or laser diode (or "diode laser")). The term "light source" may also relate to a plurality of light sources, such as 2-200 (solid state) LED light sources. Hence, the term LED may also refer to a plurality of LEDs. Further, the term "light source" may in embodiments also refer to a so-called chips-on-board (COB) light source. The term "COB" especially refers to LED chips in the form of a semiconductor chip that is neither encased nor connected but directly mounted onto a substrate, such as a PCB. Hence, a plurality of light emitting semiconductor light source may be configured on the same substrate. In embodiments, a COB is a multi LED chip configured together as a single lighting module.

The light source may have a light escape surface. Referring to conventional light sources such as light bulbs or fluorescent lamps, it may be outer surface of the glass or quartz envelope. For LED's it may for instance be the LED die, or when a resin is applied to the LED die, the outer surface of the resin. In principle, it may also be the terminal end of a fiber. The term escape surface especially relates to that part of the light source, where the light actually leaves or escapes from the light source. The light source is configured to provide a beam of light. This beam of light (thus) escapes from the light exit surface of the light source.

Likewise, a light generating device may comprise a light escape surface, such as an end window. Further, likewise a light generating system may comprise a light escape surface, such as an end window.

The term "light source" may refer to a semiconductor light-emitting device, such as a light emitting diode (LEDs), a resonant cavity light emitting diode (RCLED), a vertical cavity laser diode (VCSELs), an edge emitting laser, etc... The term "light source" may also refer to an organic light-emitting diode (OLED), such as a passive-matrix (PMOLED) or an active-matrix (AMOLED). In a specific embodiment, the light source comprises a solid-state light source (such as a LED or laser diode). In an embodiment, the light source comprises a LED (light emitting diode). The terms "light source" or "solid state light source" may also refer to a superluminescent diode (SLED).

The term LED may also refer to a plurality of LEDs.

The term "light source" may also relate to a plurality of (essentially identical (or different)) light sources, such as 2-2000 solid state light sources. In embodiments, the light source may comprise one or more micro-optical elements (array of micro lenses) downstream of a single solid-state light source, such as a LED, or downstream of a plurality of solid-state light sources (i.e. e.g. shared by multiple LEDs). In embodiments, the light source may comprise a LED with on-chip optics. In embodiments, the light source comprises a pixelated single LEDs (with or without optics) (offering in embodiments on-chip beam steering).

In embodiments, the light source may be configured to provide primary radiation, which is used as such, such as e.g. a blue light source, like a blue LED, or a green light source, such as a green LED, and a red light source, such as a red LED. Such LEDs, which may not comprise a luminescent material ("phosphor") may be indicated as direct color LEDs.

In other embodiments, however, the light source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the light source, such as a LED with a luminescent material layer or dome comprising luminescent material. Such LEDs may be indicated as phosphor converted LEDs or PC LEDs (phosphor converted LEDs). In other embodiments, the luminescent material may be configured at some distance ("remote") from the light source, such as a LED with a luminescent material layer not in physical contact with a die of the LED. Hence, in specific embodiments the light source may be a light source that during operation emits at least light at wavelength selected from the range of 380-470 nm. However, other wavelengths may also be possible. This light may partially be used by the luminescent material.

In embodiments, the light generating device may comprise a luminescent material. In embodiments, the light generating device may comprise a PC LED. In other embodiments, the light generating device may comprise a direct LED (i.e. no phosphor). In embodiments, the light generating device may comprise a laser device, like a laser diode. In embodiments, the light generating device may comprise a superluminescent diode. Hence, in specific embodiments, the light source may be selected from the group of laser diodes and superluminescent diodes. In other embodiments, the light source may comprise an LED.

The light source may especially be configured to generate light source light having an optical axis (O), (a beam shape,) and a spectral power distribution. The light source light may in embodiments comprise one or more bands, having band widths as known for lasers.

The term "light source" may (thus) refer to a light generating element as such, like e.g. a solid state light source, or e.g. to a package of the light generating element, such as a solid state light source, and one or more of a luminescent material comprising element and (other) optics, like a lens, a collimator. A light converter element ("converter element" or "converter") may comprise a luminescent material comprising element. For instance, a solid state light source as such, like a blue LED, is a light source. A combination of a solid state light source (as light generating element) and a light converter element, such as a blue LED and a light converter element, optically coupled to the solid state light source, may also be a light source (but may also be indicated as light generating device). Hence, a white LED is a light source (but may e.g. also be indicated as (white) light generating device).

The term "light source" herein may also refer to a light source comprising a solid state light source, such as an LED or a laser diode or a superluminescent diode.

The "term light source" may (thus) in embodiments also refer to a light source that is (also) based on conversion of light, such as a light source in combination with a luminescent converter material. Hence, the term "light source" may also refer to a combination of a LED with a luminescent material configured to convert at least part of the LED radiation, or to a combination of a (diode) laser with a luminescent material configured to convert at least part of the (diode) laser radiation.

In embodiments, the term "light source" may also refer to a combination of a light source, like a LED, and an optical filter, which may change the spectral power distribution of the light generated by the light source. Especially, the "term light generating device" may be used to address a light source and further (optical components), like an optical filter and/or a beam shaping element, etc.

The phrases "different light sources" or "a plurality of different light sources", and similar phrases, may in embodiments refer to a plurality of solid-state light sources selected from at least two different bins. Likewise, the phrases "identical light sources" or "a plurality of same light sources", and similar phrases, may in embodiments refer to a plurality of solid-state light sources selected from the same bin.

The term "solid state light source", or "solid state material light source", and similar terms, may especially refer to semiconductor light sources, such as a light emitting diode (LED), a diode laser, or a superluminescent diode.

Instead of the term "solid state light source" also the term "semiconductor-based light source" may be applied. Hence, the term "semiconductor-based light source" may e.g. refer to one or more of a light emitting diode (LED), a diode laser, and a superluminescent diode.

The first light generating device is configured to generate first device light having a first wavelength in the visible wavelength range.

The terms "visible", "visible light" or "visible emission" and similar terms refer to light having one or more wavelengths in the range of about 380-780 nm. Herein, UV may especially refer to a wavelength selected from the range of 190-380 nm, such as 200-380 nm.

The terms "light" and "radiation" are herein interchangeably used, unless clear from the context that the term "light" only refers to visible light. The terms "light" and "radiation" may thus refer to UV radiation, visible light, and IR radiation. In specific embodiments, especially for lighting applications, the terms "light" and "radiation" refer to (at least) visible light.

The terms "violet light" or "violet emission" especially relates to light having a wavelength in the range of about 380-440 nm. The terms "blue light" or "blue emission" especially relates to light having a wavelength in the range of about 440-495 nm (including some violet and cyan hues). The terms "green light" or "green emission" especially relate to light having a wavelength in the range of about 495-570 nm. The terms "yellow light" or "yellow emission" especially relate to light having a wavelength in the range of about 570-590 nm. The terms "orange light" or "orange emission" especially relate to light having a wavelength in the range of about 590-620 nm. The terms "red light" or "red emission" especially relate to light having a wavelength in the range of about 620-780 nm. The term "pink light" or "pink emission" refers to light having a blue and a red component. The term "cyan" may refer to one or more wavelengths selected from the range of about 490-520 nm. The term "amber" may refer to one or more wavelengths selected from the range of about 585-605 nm, such as about 590-600 nm. The phrase "light having one or more wavelengths in a wavelength range" and similar phrases may especially indicate that the indicated light (or radiation) has a spectral power distribution with at least intensity or intensities at these one or more wavelengths in the indicate wavelength range. For instance, a blue emitting solid state light source will have a spectral power distribution with intensities at one or more wavelengths in the 440-495 nm wavelength range.

Especially, the first device light may have a centroid wavelength within the wavelength range of 380-780 nm.

The term "centroid wavelength", also indicated as λc, is known in the art, and refers to the wavelength value where half of the light energy is at shorter and half the energy is at longer wavelengths; the value is stated in nanometers (nm). It is the wavelength that divides the integral of a spectral power distribution into two equal parts as expressed by the formula λc = Σ λ*I(λ) / (Σ I(λ)), where the summation is over the wavelength range of interest, and I(λ) is the spectral energy density (i.e. the integration of the product of the wavelength and the intensity over the emission band normalized to the integrated intensity). The centroid wavelength may e.g. be determined at operation conditions.

In embodiments, at least 80%, more especially at least 90%, even more especially at least 95%, of the spectral power (in Watt) of the first device light may be in the visible wavelength range.

In embodiments, the second light generating device may be configured to generate second device light having a second wavelength in the UV wavelength range. As indicated above, the second wavelength may be in the 100-380 nm wavelength range, such as especially as especially 190-380 nm. Especially, the second device light may have a centroid wavelength in the 190-380 nm wavelength range. The first device light and the second device light may have centroid wavelengths differing at least 15 nm, such as at least 20 nm, like in specific embodiments at least 25 nm. For instance, in embodiments the first device light and the second device light may have centroid wavelengths differing at least 30 nm. However, smaller values may also be possible.

In specific embodiments, the second light generating device may be configured to generate second device light having one or more second wavelengths in the UV-B and/or UV-C wavelength ranges. For instance, in embodiments the second light generating device may be configured to generate second device light having one or more second wavelengths in the UV-B and/or near-UV-C wavelength ranges.

In embodiments, at least 80%, more especially at least 90%, even more especially at least 95%, of the spectral power (in Watt) of the first device light may be in the 100-380 nm wavelength range, more especially in the 190-380 nm wavelength range.

Further, as indicated above the light generating system comprises a first diffuser and a second diffuser.

Here below, some general embodiments relating to diffusers are described, which may individually apply to the first diffuser and the second diffuser.

Instead of the term "diffuser" also the terms "light diffuser" or "optical diffuser" may be applied. Especially, a diffuser may be a material that diffuses or scatters light. As can be derived from https://www.rp-photonics.com/diffusers.html, an optical diffuser is a device which can diffuse light. This may imply scrambling its wavefronts and reducing its spatial coherence. In other words, one may obtain random or pseudo-random changes of optical phase for different parts of the special profile of incoming light. For example, if a highly spatially coherent laser beam hits a diffuser, the light emerging from the diffuser may no longer have the characteristics of a beam, but rather propagate in a (wide) range of directions. In most, but not all cases, a diffuser works based on light scattering or refraction on or in a stationary piece of material with a highly random structure. For instance, one can use diffuse scattering on white ceramics or a sandblasted (and thus micro structured) optical surface in a reflective geometry. Some devices have a scattering medium on top of a mirror surface, e.g. a protected metal-coated mirror. In simple cases, even a piece of white paper is sufficient. Alternatively, one can transmit light through a piece of ground, sandblast or chemically etched glass (also called frosted glass or milk glass) or a photopolymer, containing many scattering centers. In some cases, one uses glass or plastic optics with structured surfaces, such that one obtains refraction as on small prisms. Holographic diffusers can have particularly well controlled scattering properties. There are also transmissive or reflective micro-optical diffusers, containing a pseudo-random structure, where each part of an incident beam experiences a quasi-random change of optical phase. Such devices often function as holographic diffusers, where each light beam obtains a (quasi-)random phase change, but does not undergo multiple random scattering processes. The scattered light distribution can be controlled by using a suitable design of the holographic pattern. For example, one can achieve a given angular distribution of transmitted light while largely avoiding any back-reflection. One may exploit random light scattering in a liquid or gas containing scattering centers with an appropriate density. Random optical diffusers often contain scattering centers with roughly circular shape, or sometimes with very random shapes. For example, frosted glass may contain tiny air bubbles, where the strong refractive index contrast between air and glass leads to substantial scattering. One may distinguish between reflective diffusers (= backward scattering diffusers) and transmissive diffusers (= forward scattering diffusers). The former often surface diffusers, where scattering occurs on the surface of an opaque material, while transmissive diffusers may be volume diffusers, where the scattering occurs within the volume of a transparent medium, or also exploit scattering at surfaces. Some diffusers are semi-opaque, i.e., part of the light is transmitted, while another part is diffusely reflected.

Especially, the diffuser is a transmissive diffuser, more precisely a translucent diffuser. Hence, light may be transmitted but at least part of the transmitted light is scattered. Light having a non-Lambertian distribution propagating through a diffuser may have a more Lambertian-like distribution downstream of the diffuser. In embodiments, the diffuser may comprise a transparent material with particles embedded therein. Alternatively or additionally, the diffuser may comprise a transparent material with surface elements that scatter the light. In embodiments, the diffuser may comprise a diffractive diffuser.

The diffuser may transmit part of the light that is received by the diffuser but may also reflect part of the light that is received by the diffuser. Hence, the diffuser may be a semi-opaque diffuser.

The term "diffuser" may also refer to a plurality of (the same or different) diffusers.

In the present invention, there are at least two diffusers, which are configured at different positions. Further, also the different light generating devices are configured at different positions. Hence, the light generating system comprises at least two different diffusers which may receive light having different spectral power distributions.

The first light generating device is configured upstream of the first diffuser and upstream of the second diffuser. Hence, the first device light may only escape from the system via the first diffuser and the second diffuser.

The second light generating device is configured downstream of the first diffuser and upstream of the second diffuser. Hence, the second device light may escape from the system via only the second diffuser. Hence, a substantial percentage of the second device light that escapes from the system may escape from the system via the second diffuser only (and not via transmissions through the first diffuser, followed by transmission by the second diffuser)(see also below). The phrase "the second light generating device may be configured downstream of the first diffuser and upstream of the second diffuser", and similar phrases, may especially indicate that the second light generating device(s) may be configured between the first diffuser and the second diffuser.

Scattering may be provided by particles embedded in the (light transmissive) material of the diffuser. Such particles may be scattering particles (like e.g. comprising one or more of Al₂O₃, BaSO₄ and TiO₂). In this way, a light transmissive body may be a diffuser.

Scattering may also be provided via scattering features at one or more faces of light transmissive body, like indentations, scratches, grooves, dots of material, light scattering structures (in optical contact with one of the faces), etc. etc. In this way, a light transmissive body may be a diffuser.

In specific embodiments, the second diffuser may be comprises by an end window or may be an end window. In other embodiments, optics may be configured downstream of the second diffuser.

The terms "upstream" and "downstream" relate to an arrangement of items or features relative to the propagation of the light from a light generating means (here the especially the light source), wherein relative to a first position within a beam of light from the light generating means, a second position in the beam of light closer to the light generating means is "upstream", and a third position within the beam of light further away from the light generating means is "downstream".

In embodiments, one of the first diffuser and the second diffuser may be a back scattering diffuser. In embodiments, of one of the first diffuser and the second diffuser, the percentage of back scattering may be larger than the percentage of forward scattering.

In embodiments, (another) one of the first diffuser and the second diffuser may be a forward scattering diffuser. In embodiments, of one of the first diffuser and the second diffuser, the percentage of forward scattering may be larger than the percentage of back scattering.

Back scattering may be promoted by scattering particles in the bulk material of the diffuser. Forward scattering may be promoted by scattering features at a face of the diffuser.

In embodiments, one of the diffusers essentially only comprises scattering particles embedded in the material of the diffuser. This may especially apply to the first diffuser. Alternatively or additionally, in embodiments, (another) one of the diffusers essentially only comprises scattering features at a surface of the diffuser. This may especially apply to the second diffuser.

The amount of backscattering and forward scattering can be measured, for instance with a spectrometer with an integrating sphere. Persons skilled in the art know how to perform such measurements.

As indicated above, the first device light may only escape from the system via the first diffuser and the second diffuser. The first diffuser and the second diffuser are transmissive for the first device light. Further, the first diffuser and the second diffuser are diffusive for the first device light. Therefore, the first diffuser and the second diffuser are (i) transmissive for the first device light and (ii) diffusive for the first device light.

The second diffuser is not only be transmissive for the first device light but is also transmissive for the second device light. Further, in embodiments the second diffuser may (also) be diffusive for the second device light. For instance, the second diffuser may comprise a light transmissive material, such as quarts, transmissive for both the first device light and the second device light, including scattering structures to scatter the first device light and optionally also the second device light.

Herein, transmission and reflection values for the diffuser may be refer to values obtainable under perpendicular radiation.

The first diffuser has a first UV reflection R21 for the second device light. Further the second diffuser has a second UV reflection R22 for the second device light.

Especially, it may be desirable that the second device light is reflected by the first diffuser and transmission of the first diffuser for the second device light may in embodiments be relatively low. However, transmission of the second diffuser for the second device light may be higher than transmission of the second device light by the first diffuser. Further, it appears beneficial when the reflection of the second diffuser for the second light is smaller than the reflection of the second device light by the first diffuser. Therefore, according to the invention, R21 is larger than R22 (i.e. R21>R22).

Especially, in embodiments (R21-R22)≥5%, such as (R21-R22)≥10% may apply. In embodiments, 10%≤(R21-R22)≤40%. However, other values are herein not excluded. With these values, the second device light may be facilitated to escape from the system via the second diffuser. Further, with these values, a possible loss of the second device light due to transmission through the first diffuser may be minimized.

Further, desirable values for the first UV reflection R21 by the first diffuser may be selected from the range of 10%≤R21≤70%, more especially 20%≤R21≤60%. In embodiments, 25%≤R21≤50%. Yet further, desirable values for the second UV reflection R22 by the second diffuser may be selected from the range of 5%≤R22≤30%, more especially 10%≤R22≤25%. In embodiments, 10%≤R22≤20% may apply. Hence, in specific embodiments 20%≤R21≤60% and 10%≤R22≤20% may apply. The reflection of the second device light by the first diffuser and second diffuser is according to the invention selected from the range of 25%≤(R21+R22)≤75%. More especially, in embodiments 30%≤(R21+R22)≤70% may apply.

Further, desirable values for the first visible light reflection R11 by the first diffuser may be selected from the range of 5%≤R11≤70%, such as 10%≤R11≤70%, like in embodiments 20%≤R11≤60%. In embodiments, 25%≤R11≤50%. Yet further, desirable values for the first visible light reflection R12 by the second diffuser may be selected from the range of 5%≤R12≤30%, more especially 10%≤R12≤25%. In embodiments, 10%≤R12≤20% may apply. Hence, in specific embodiments 20%≤R11≤60% and 10%≤R12≤20% may apply.

Further, the reflection of the first device light by the first diffuser and second diffuser may in embodiments be selected from the range of 25%≤(R11+R12)≤75%. More especially, in embodiments 30%≤(R11+R12)≤70% may apply.

The first diffuser may have a first transmission for the first device light T11 and the second diffuser may have a second transmission for the first device light T12.

Especially T12>T11.

The first diffuser may have a first transmission for the second device light T21 and the second diffuser may have a second transmission for the second device light T22.

Especially T11>T21.

Especially, T11 may be at least 40%, such as at least about 50%, more especially at least about 60%, such as at least about 70%, like in embodiments at least about 80%.

Especially, T21 may be at maximum 50%, such as at maximum 40%, like at maximum 30%.

Especially, T12 may be at least 40%, such as at least about 50%, more especially at least about 60%, such as at least about 70%, like in embodiments at least about 80%.

The transmission of the first device light by the first diffuser and the second diffuser may have a minimum value. For instance, at least 25% of the first device light may escape from the second diffuser. In embodiments, (T11*T12/10000)≥0.25, such as (T11*T12/10000)≥0.3, more especially (T11*T12/10000)≥0.35.

Especially, T22 may be at least 30%, such as at least about 40%, more especially at least about 50%, such as at least about 60%. In embodiments T22 may be equal to or smaller than 95%, such as equal to or smaller than 90%.

The first number after T or R may refer to the first device light when 1 is used and to the second device light when 2 is used. The second number after T or R may refer to the first diffuser when 1 is used and to the second diffuser when 2 is used. Hence, for instance R21 refers to the reflection of the second device light by the first diffuser.

Hence, in embodiments the first diffuser is essentially only configured downstream of the first light generating devices and the second diffuser is essentially configured downstream of both the first light generating devices and the second light generating devices.

In an operational mode (of the light generating system), the light generating system is configured to generate system light comprising one or more of the first device light and the second device light. For instance, in an operational mode (of the light generating system) both the first device light and the second device light may be provided. When the first light generating device and the second light generating device are controllable, it may also be possible to provide only one of the first device light and the second device light. Hence, in embodiments in an operational mode (of the light generating system) the light generating system may be configured to generate system light comprising the first device light (and not the second device light). Alternatively, in embodiments in another operational mode (of the light generating system) the light generating system may be configured to generate system light comprising the second device light (and not the first device light).

Hence, the light generating system may further comprise a control system. Especially, the control system may be configured to control the first light generating device and the second light generating device. In this way, the spectral power distribution of the system light may be controlled. Hence, in embodiments the spectral power distribution in the range of 190-780 nm may be controlled.

The term "controlling" and similar terms especially refer at least to determining the behavior or supervising the running of an element. Hence, herein the term "controlling", and similar terms, may e.g. refer to imposing behavior to the element (determining the behavior or supervising the running of an element), etc., such as e.g. measuring, displaying, actuating, opening, shifting, changing temperature, etc. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions from a remote control. In embodiments, the control system may be controlled via an App on a device, such as a portable device, like a Smartphone or I-phone, a tablet, etc. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, WIFI, LiFi, ZigBee, BLE or WiMAX, or another wireless technology.

The system, or apparatus, or device may execute an action in a "mode" or "operation mode" or "mode of operation" or "operational mode". The term "operational mode may also be indicated as "controlling mode". Likewise, in a method an action or stage, or step may be executed in a "mode" or "operation mode" or "mode of operation" or "operational mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

The light generating system comprises two light mixing chambers. In one of the chambers, the first device light is provided. In the other of the chamber, the second device light is provided. The latter chamber is configured downstream of the former. When using light mixing chamber, except for a light exit window, a substantial part of the walls may be reflective for the light generated in the chamber.

The first light mixing chamber comprises a first chamber enclosure, wherein the first chamber enclosure comprises the first diffuser and a first reflector, wherein the first light generating device is at least partially enclosed by the first chamber enclosure. Especially, a light emitting surface of the first light generating device may be configured within the first light mixing chamber. The second light mixing chamber comprises a second chamber enclosure, wherein the second chamber enclosure comprises the second diffuser and a second reflector, wherein the second light generating device is at least partially enclosed by the second chamber enclosure. Therefore, the light generating system comprises a first light mixing chamber and a second light mixing chamber; wherein: (a) the first light mixing chamber comprises a first chamber enclosure, wherein the first chamber enclosure comprises the first diffuser and a first reflector, wherein the first light generating device is at least partially enclosed by the first chamber enclosure; and (b) the second light mixing chamber comprises a second chamber enclosure, wherein the second chamber enclosure comprises the second diffuser and a second reflector, wherein the second light generating device is at least partially enclosed by the second chamber enclosure. Especially, in embodiments the second chamber enclosure may (also) comprises the first diffuser. Hence, the second chamber enclosure may comprise two light transmissive windows: one may be provided by the first diffuser and another one may be provided by the second diffuser. The term "first reflector" may also refer to a plurality of first reflectors. The term "first reflector" may also refer to a plurality of first reflectors.

In specific embodiments, the first light mixing chamber and the second light mixing chamber are stacked.

For instance, both light mixing chamber may have a circular, rectangular, or hexagonal cross-section. However, other cross-sectional shapes may also be possible. Rectangular shapes may be useful when an array of light generating systems (or lighting devices) may be applied.

In embodiments, the light mixing chamber may be tapering in a direction from the second light mixing chamber to the first light mixing chamber.

In embodiment, the first diffuser may have a first cross-sectional area A1 and the second diffuser may have a second cross-sectional area A2. The cross-sectional areas may be determined perpendicular to an optical axis of the light generating system and/or perpendicular to one or both diffusers. In embodiments A1=A2 (see also below). In other embodiments, however, A2=x*A1, wherein x may be selected from the range of 1.2-16, such as selected from the range of 1.5-4, like in embodiments at least 1.8, or even at least 2.

In other embodiments, the cross-sectional dimensions of the first light mixing chamber and the second light mixing chamber may stay essentially the same over the height (of the combination of the first light mixing chamber and the second light mixing chamber). Hence, in such embodiments A1 may essentially the same as A2.

In (other) specific embodiments the second light mixing chamber may at least partially encloses the first light mixing chamber.

For instance, the first light mixing chamber and the second light mixing chamber may have a U-like the cross-sectional shape, with the former having smaller dimensions than the latter. A perpendicular cross-sectional shape may be circular, rectangular, or hexagonal, especially circular.

In embodiments, the first light mixing chamber may have a circular, rectangular, or hexagonal, especially circular, cross-sectional shape, and the second light mixing chamber may have a circular, rectangular, or hexagonal, especially circular, cross-sectional shape, which shapes may be different, but which may especially be the same, wherein the second light mixing chamber has such dimension, that the first light mixing chamber may be at least partially enclosed by the second light mixing chamber. For instance, a larger cylinder may enclose a smaller cylinder, or a larger dome may enclose a smaller dome, etc.

In embodiments, the first diffuser may be substantially planar and the second diffuser may be curved (in a plane enclosing the optical axis of the system). In other embodiments, the second diffuser may be substantially planar and the first diffuser may be curved (in a plane enclosing the optical axis of the system).

Especially, the first diffuser and the second diffuser may share an axis that is perpendicular to both first diffuser and the second diffuser. In embodiments, an optical axis of the system may intercept a middle of the first diffuser and a middle of the second diffuser.

As indicated above, the diffuser may especially be light transmissive diffusers.

The first light generating device and the second light generating device may be directed in the same direction, i.e. with colinear optical axis or with a mutual angle between the optical axes. The mutual angle may be small or may be in the order of perpendicular. As light mixing chamber are used, the direction of the optical axes may be less important, though especially the optical axes do not have an angle larger than about 90° with an optical axis of the light generating device. Hence, in embodiments the optical axes of the first light generating device and the second light generating device may especially be found with a hemisphere.

Especially, the first light generating device may have first optical axis (O1), the second light generating device may have a second optical axis (O2).

In embodiments, the first optical axis (O1) and the second optical axis (O2) may have a mutual angle α12 selected from the range of 15-90°. Alternatively, in embodiments the first optical axis (O1) and the second optical axis (O2) have a mutual angle α12 selected from the range of 0-15°. Mutual angles larger than 15°, such as in the order of 45° or larger, may add to the homogeneity of the visible system light. This may especially apply when a second angle α2 with an optical is (also) selected from the range of 15-90°, more especially 45-90°.

As indicated above, the first light generating device may comprise one or more first light generating devices and/or the second light generating device may comprise one or more second light generating devices. In specific embodiments, the light generating system may comprise n1 first light generating devices and n2 second light generating devices, wherein in specific embodiments n2<n1. In embodiments, n1=1. In other embodiments, n1 may be selected from the range of at least 2, such as at least 4. In embodiments, n2=2. In other embodiments, n2 may be selected from the range of at least 3, such as at least 8. In embodiments, n2/n1≤1/3. However, other ratios are herein not excluded.

In specific embodiments, the n1 first light generating devices and n2 second light generating devices may be configured in an array, wherein three or more first light generating devices enclose one or more second light generating devices; and wherein in further specific embodiments n2/n1≤1/3.

Alternatively, the n1 first light generating devices and n2 second light generating devices are configured in an array wherein the first light generating devices and the second light generating devices are configured interleaved. In this way, possible black spot formation may be minimized.

As indicated above, in embodiments the first light generating devices and the second light generating devices comprise solid-state light sources.

The system may be configured to provide in an operational mode white system light. The term "white light", and similar terms, herein, is known to the person skilled in the art. It may especially relate to light having a correlated color temperature (CCT) between about 1800 K and 20000 K, such as between 2000 and 20000 K, especially 2700-20000 K, for general lighting especially in the range of about 2000-7000 K, such as in the range of 2700 K and 6500 K. In embodiments, e.g. for backlighting purposes, or for other purposes, the correlated color temperature (CCT) may especially be in the range of about 7000 K and 20000 K. Yet further, in embodiments the correlated color temperature (CCT) is especially within about 15 SDCM (standard deviation of color matching) from the BBL (black body locus), especially within about 10 SDCM from the BBL, even more especially within about 5 SDCM from the BBL. In specific embodiments, the correlated color temperature (CCT) may be selected from the range of 6000-12000 K, like selected from the range of 7000-12000 K, like at least 8000 K. Yet further, in embodiments the correlated color temperature (CCT) may be selected from the range of 6000-12000 K, like selected from the range of 7000-12000 K, in combination with a CRI of at least 70.

Especially, in embodiments the first light generating device may be configured to generate white first device light having a correlated color temperature in a range from 2000 to 8000 K and a color rendering index of at least 80, and the second light generating device may be configured second device light having one or more second wavelengths in the UV-B and/or UV-C wavelength ranges, such as one or more second wavelengths in the UV-B and/or near-UV-C wavelength ranges.

As indicated above, the system may comprise a control system. Further, the system may comprise a sensor. The first device light and/or the second device light may be controlled in dependence of a sensor signal of the sensor. Hence, in embodiments the system may further comprise a control system and a sensor, wherein the control system may be configured to control the first light generating device and the second light generating device in dependence of a sensor signal of the sensor.

A sensor signal is the sensor signal of a sensor. The term "sensor" may also refer to a plurality of (different) sensors.

In embodiments, the sensor may be selected from the group comprising a movement sensor, a presence sensor, a distance sensor, an ion sensor, a gas sensor, a volatile organic compound sensor, a pathogen sensor, an airflow sensor, a sound sensor, a temperature sensor, and a humidity sensor.

A movement sensor may be used to sense people. A movement sensor may also be used to sense the number of people. A movement sensor may also be used to sense an activity level of the people (e.g. occupied or non-occupied working cubicle or fitness room).

A presence sensor may be used to sense people. A presence sensor may also be used to sense the number of people. A presence sensor may also be used to sense an activity level of the people (e.g. occupied or non-occupied working cubicle or fitness room). A distance sensor may be used to sense one or more dimensions of a space for which the ionizer device is used. A distance sensor may also be used to sense distances between people. The ion sensor may comprise a positive ion sensor. Additionally or alternatively, the ion sensor may comprise a negative ion sensor. The ion sensor may be used to sense the effect of the ionizer device (the more ions, the better the air treatment may be). A gas sensor may be used to sense gas one or more gas components. The gas sensor may be used to sense whether ventilation is sufficient or insufficient. The gas sensor may e.g. (thus) also be used to sense the number of people and/or an activity level of the people. A volatile organic compound (VOG) sensor may be used to sense one or more volatile organic compounds. The VOG sensor may be used to sense whether ventilation is sufficient or insufficient. The VOG sensor may e.g. (thus) also be used to sense the number of people and/or an activity level of the people. The pathogen sensor may comprise a sensor for one or more of bacteria, viruses, and spores. The pathogen sensor may be used to sense whether ventilation is sufficient or insufficient. The pathogen sensor may e.g. (thus) also be used to sense the number of people and/or an activity level of the people. An airflow sensor may be used to sense an airflow. The airflow sensor may be used to sense whether ventilation is sufficient or insufficient. The airflow sensor may e.g. (thus) also be used to sense the number of people and/or an activity level of the people. A sound sensor may be used to sense sound. The sound sensor may be used to sense whether ventilation is sufficient or insufficient. The sound sensor may e.g. (thus) also be used to sense the number of people and/or an activity level of the people. The temperature sensor may be used to sense temperature. On the basis thereon, it may be determined whether pathogens may be more detrimental or less detrimental. The humidity sensor may be used to sense (air) humidity. On the basis thereon, it may be determined whether pathogens may be more detrimental or less detrimental (as there seems to be a relation between humidity and transferability of e.g. airborne pathogens). In specific embodiments, the sensor may comprise one or more sensors selected from the group of a daylight sensor, a proximity sensor, a movement sensor, a presence sensor, a CO₂ sensor, a virus sensor, and a VOC sensor.

The light generating system may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, (outdoor) road lighting systems, urban lighting systems, green house lighting systems, horticulture lighting, digital projection, or LCD backlighting. The light generating system (or luminaire) may be part of or may be applied in e.g. optical communication systems or disinfection systems.

In yet a further aspect, the invention also provides a lamp or a luminaire comprising the light generating system as defined herein. The luminaire may further comprise a housing, optical elements, louvres, etc. etc. The lamp or luminaire may further comprise a housing enclosing the light generating system. The lamp or luminaire may comprise a light window in the housing or a housing opening, through which the system light may escape from the housing. In yet a further aspect, the invention also provides a projection device comprising the light generating system as defined herein. Especially, a projection device or "projector" or "image projector" may be an optical device that projects an image (or moving images) onto a surface, such as e.g. a projection screen. The projection device may include one or more light generating systems such as described herein. Hence, in an aspect the invention also provides a light generating device selected from the group of a lamp, a luminaire, a projector device, a disinfection device, a photochemical reactor, and an optical wireless communication device, comprising the light generating system as defined herein. The light generating device may comprise a housing or a carrier, configured to house or support, one or more elements of the light generating system. For instance, in embodiments the light generating device may comprise a housing or a carrier, configured to support the first light generating device and/or the second light generating device.

In a further aspect, the invention also provides a method for one or more of (i) treating a gas, especially air, or a surface in a space, and (ii) providing light in the space; wherein: (a) the space is external from the light generating system as defined herein or external from the lighting device as defined herein; and (ii) the method comprises providing system light in the space (to the gas or the surface with the light generating system).

The term "space" may for instance relate to a (part of) hospitality area, such as a restaurant, a hotel, a clinic, or a hospital, etc.. The term "space" may also relate to (a part of) an office, a department store, a warehouse, a cinema, a church, a theatre, a library, etc. However, the term "space" may also relate to (a part of) a working space in a vehicle, such as a cabin of a truck, a cabin of an air plane, a cabin of a vessel (ship), a cabin of a car, a cabin of a crane, a cabin of an engineering vehicle like a tractor, etc. The term "space" may also relate to (a part of) a working space, such as an office, a (production) plant, a power plant (like a nuclear power plant, a gas power plant, a coal power plant, etc.), etc. For instance, the term "space" may also relate to a control room, a security room, etc. Especially, the term "space" may herein refer to an indoor space. In yet other embodiments, the term "space" may also relate to a toilet room or bathroom. In yet other embodiments, the term "space" may also relate to an elevator. In embodiments, the term "space" may also refer to a conference room, a school room, an indoor hallway, an indoor corridor, an indoor space in an elderly home, an indoor space in a nursing home, etc. In embodiments, the term "space" may refer to an indoor sport space, like a gym, a gymnastics hall, in indoor ball sport space, a ballet room, a swimming pool, a changing room, etc. In embodiments, the term "space" may refer to an (indoor) bar, an (indoor) disco, etc.

In embodiments, a plurality of lighting devices may be applied as defined herein and one or more other lighting devices may be applied, having essentially the same shape and dimensions. The herein described lighting devices may e.g. be combined with lighting devices essentially configured to generate only visible light. For instance, in a space a grid of lighting devices may be provided, of which one or more are as defined herein, and of which one or more are not according to the invention. Hence, a modular approach may be possible with the present invention.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1b schematically depict some embodiments; and
Fig. 2 schematically depict some applications. The schematic drawings are not necessarily to scale.

### DETAILED DESCRIPTION OF THE EMBODIMENTS

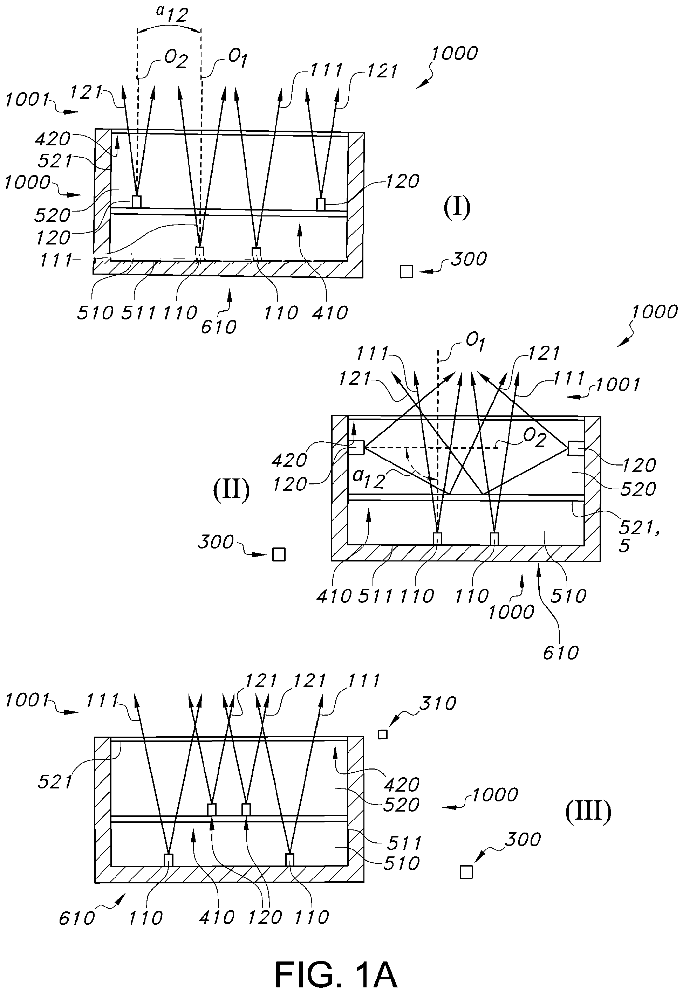

Fig. 1a schematically depicts some embodiments of a light generating system 1000 comprising a first light generating device 110, a second light generating device 120, a first diffuser 410, and a second diffuser 420.

The first light generating device 110 is configured to generate first device light 111 having a first wavelength in the visible wavelength range. Especially, the first light generating device 110 may comprise a solid-state light source, such as a LED, a superluminescent diode, or a laser diode. The first light generating device 110 is configured upstream of the first diffuser 410 and upstream of the second diffuser 420.

The second light generating device 120 may be configured to generate second device light 121 having a second wavelength in the UV wavelength range. Especially, the second light generating device 120 may comprise a solid-state light source, such as a LED, a superluminescent diode, or a laser diode. The second light generating device 120 is configured downstream of the first diffuser 410 and upstream of the second diffuser 420. As schematically depicted, the second light generating device 120 may be configured between the first diffuser 410 and the second diffuser 420.

Hence, in embodiments the first light generating devices 110 and the second light generating devices 120 may comprise solid-state light sources.

The first diffuser 410 and the second diffuser 420 are (i) transmissive for the first device light 111 and (ii) diffusive for the first device light 111. Further, the second diffuser 420 is transmissive for the second device light 121 (and may also be diffusive for the second device light 121).

The first diffuser 410 has a first UV reflection R21 for the second device light 121. The second diffuser 420 has a second UV reflection R22 for the second device light 121. R21 is larger than R22.

The light generating system 1000 is configured to generate system light 1001 comprising one or more of the first device light 111 and the second device light 121.

In specific embodiments, (R21-R22)≥10% may apply. In specific embodiments, 30%≤(R21+R22)≤70% may apply. In specific embodiments, 20%≤R21≤60% and/or 10%≤R22≤20% may apply.

In embodiments, one of the diffusers essentially only comprises scattering particles embedded in the material of the diffuser. This may especially apply to the first diffuser. Alternatively or additionally, in embodiments, (another) one of the diffusers essentially only comprises scattering features at a surface of the diffuser. This may especially apply to the second diffuser.

As schematically depicted in Fig. 1a, the light generating system 1000 comprises a first light mixing chamber 510 and a second light mixing chamber 520.

The first light mixing chamber 510 comprises a first chamber enclosure 511. The first chamber enclosure 511 comprises the first diffuser 410 and a first reflector 450. The first light generating device 110 is at least partially enclosed by the first chamber enclosure 511.

The second light mixing chamber 520 comprises a second chamber enclosure 521. The second chamber enclosure 521 comprises the second diffuser 420 and a second reflector 460. The second light generating device 120 is at least partially enclosed by the second chamber enclosure 521.

As schematically depicted, the second chamber enclosure 521 may (also) comprise the first diffuser 410. Further, as schematically depicted, the first light mixing chamber 510 and the second light mixing chamber 520 may be stacked.

Referring to Figs. 1a (and 1b) second device light that escapes from the system may escape from the system via essentially the second diffuser only (and not via a detour via transmissions through the first diffuser, followed by transmission by the second diffuser). First device light, however, may only escape from the system via the first diffuser and subsequently via the second diffuser. For both may apply that a part may be reflected at the diffuser, which may, after some reflections, again reach one of the diffusers.

Reference 610 refers to a support, such as a PCB or a support comprising a PCB and a PCB support, such as a PCB functionally coupled to a heatsink.

The first light generating device 110 may have first optical axis O1 and the second light generating device 120 may have a second optical axis O2. In embodiments, the first optical axis O1 and the second optical axis O2 may have a mutual angle α12 selected from the range of 15-90°, see embodiment II. In (other) embodiments, the first optical axis O1 and the second optical axis O2 may have a mutual angle α12 selected from the range of 0-15°, see embodiments I and III. In a variation (not depicted) on embodiment II, also the first light generating devices 110 may be configured at a side face. Also in such embodiments, the first optical axis O1 and the second optical axis O2 may have a mutual angle α12 selected from the range of 0-15°.

The light generating system 1000 may further comprising a control system 300. The control system may be configured to control the first light generating device 110 and the second light generating device 120.

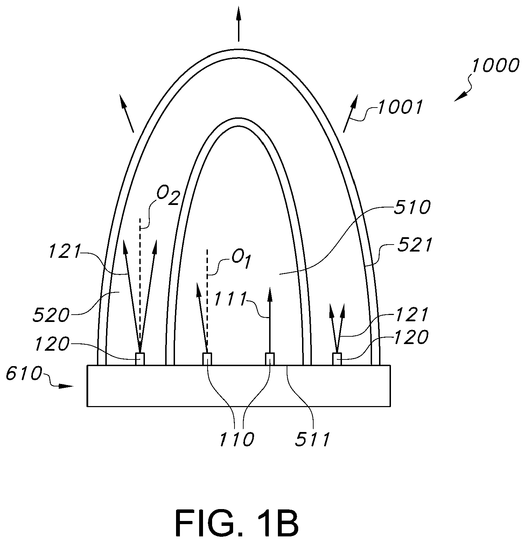

Referring to Fig. 1b, in embodiments the second light mixing chamber 520 may at least partially enclose the first light mixing chamber 510.

In specific embodiments, the light generating system 1000 may comprise n1 first light generating devices 110 and n2 second light generating devices 120. Especially, n2<n1may apply. In specific embodiments, n2/n1≤1/3 may apply.

In specific embodiments, n1 first light generating devices 110 and n2 second light generating devices 120 may be configured in an array. In further specific embodiments, three or more first light generating devices 110 enclose one or more second light generating devices 120. In (other) embodiments, the first light generating devices 110 and the second light generating devices 120 are configured interleaved.

In specific embodiments, the first light generating device 110 may be configured to generate white first device light 111 having a correlated color temperature in a range from 2000 to 8000 k and a color rendering index of at least 70, such as at least 80, and/or the second light generating device 120 may be configured second device light 121 having one or more second wavelengths in the UV-B and/or UV-C wavelength ranges.

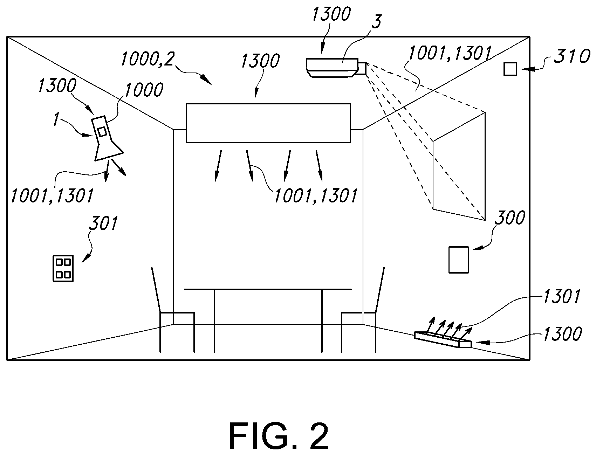

Fig. 2 schematically depicts an embodiment of a luminaire 2 comprising the light generating system 1000 as described above. Reference 301 indicates a user interface which may be functionally coupled with the control system 300 comprised by or functionally coupled to the light generating system 1000. Fig. 2 also schematically depicts an embodiment of lamp 1 comprising the light generating system 1000. Reference 3 indicates a projector device or projector system, which may be used to project images, such as at a wall, which may also comprise the light generating system 1000. Hence, Fig. 2 schematically depicts embodiments of a lighting device 1300 selected from the group of a lamp 1, a luminaire 2, a projector device 3, a disinfection device, a photochemical reactor, and an optical wireless communication device, comprising the light generating system 1000 as described herein. In embodiments, such lighting device may be a lamp 1, a luminaire 2, a projector device 3, a disinfection device, or an optical wireless communication device. Lighting device light escaping from the lighting device 1300 is indicated with reference 1201. Lighting device light 1201 may essentially consist of system light 1001, and may in specific embodiments thus be system light 1001. Light escaping from the lighting device 1300 may be indicated as device light 1301. The device light 1301 of the lighting device may essentially consist of system light 1001.

Reference 310 schematically refers to a sensor.

In specific embodiments, the light generating system 1000 may further comprising a control system 300 and a sensor 310. The control system may be configured to control the first light generating device 110 and the second light generating device 120 in dependence of a sensor signal of the sensor 310. In embodiments, the sensor 310 may comprise one or more sensors selected from the group of a daylight sensor, a proximity sensor, a movement sensor, a presence sensor, a CO₂ sensor, a virus sensor, and a VOC sensor.

Hence, the invention also provides a method for one or more of (i) treating a gas or a surface in a space, and (ii) providing light in the space. The space may be external from the light generating system 1000 or (external from) the lighting device 1300. The method may comprise providing system light 1001 in the space (to the gas or the surface with the light generating system 1000). Especially, the gas may in embodiments be air.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

It appears desirable to protect yourself and others from the spread of bacteria and viruses such as influenza or against the outbreak of novel viruses like the recent COVID-19. UV light emitted by a UV light source can be used for disinfection. Amongst others, it is herein proposed to provide a hybrid UV - white light source having a relatively high UV efficiency. Amongst others, a light generating system configured to provide system light comprising white light and UV light, is herein proposed, which may in embodiments comprise a first source of light configured to provide white light and a second source of light configured to provide UV light; a first diffuser having a first reflectance arranged downstream of the white light source and upstream of the UV light source, the first diffuser is configured to diffuse the white light; a second diffuser having a second reflectance arranged downstream of the UV light source; wherein the device light exiting the lighting device comprises white light and UV light; and the second reflectance m ay be smaller than the first reflectance. Although a combination of two diffusers may have a lower efficiency for the white light compared to using a single diffuser, the efficiency for the UV light surprisingly appears to be improved. The outer diffuser may also provide ingress protection for and/or hides the source of UV light.

## Claims

1. A light generating system (1000) comprising a first light generating device (110), a second light generating device (120), a first diffuser (410), a second diffuser (420), a first light mixing chamber (510) and a second light mixing chamber (520); wherein:
- the first light generating device (110) is configured to generate first device light (111) having a first wavelength in the visible wavelength range; wherein the first light generating device (110) is configured upstream of the first diffuser (410) and upstream of the second diffuser (420);
- the second light generating device (120) is configured to generate second device light (121) having a second wavelength in the UV wavelength range or the violet wavelength range; wherein the second light generating device (120) is configured downstream of the first diffuser (410) and upstream of the second diffuser (420);
- the first diffuser (410) and the second diffuser (420) are (i) transmissive for the first device light (111) and (ii) diffusive for the first device light (111);
- the second diffuser (420) is transmissive for the second device light (121);
- the first diffuser (410) has a first UV reflection R21 for the second device light (121); the second diffuser (420) has a second UV reflection R22 for the second device light (121), wherein R21 is larger than R22, wherein 25% ≤ (R21+R22) ≤ 75% applies; and
- the light generating system (1000) is configured to generate system light (1001) comprising one or more of the first device light (111) and the second device light (121);
- the first light mixing chamber (510) comprises a first chamber enclosure (511), wherein the first chamber enclosure (511) comprises the first diffuser (410) and a first reflector (450), wherein the first light generating device (110) is at least partially enclosed by the first chamber enclosure (511);
- the second light mixing chamber (520) comprises a second chamber enclosure (521), wherein the second chamber enclosure (521) comprises the second diffuser (420) and a second reflector (460), wherein the second light generating device (120) is at least partially enclosed by the second chamber enclosure (521);
- wherein the second light mixing chamber (520) is configured downstream of the first light mixing chamber (510).

2. The light generating system (1000) according to claim 1, wherein (R21-R22)≥10% applies.

3. The light generating system (1000) according to any one of the preceding claims, wherein 30%≤(R21+R22)≤70% applies.

4. The light generating system (1000) according to any one of the preceding claims, wherein 20%≤R21≤60% and 10%≤R22≤20% applies.

5. The light generating system (1000) according to any one of the preceding claims, wherein the UV wavelength range is in the wavelength range from 100 to 380 nm.

6. The light generating system (1000) according to any one of the preceding claims, wherein the first light mixing chamber (510) and the second light mixing chamber (520) are stacked.

7. The light generating system (1000) according to claim 5, wherein the second light mixing chamber (520) at least partially encloses the first light mixing chamber (510).

8. The light generating system (1000) according to any one of the preceding claims 1-7, wherein the first light generating device (110) has first optical axis (O1), wherein the second light generating device (120) has a second optical axis (O2), wherein the first optical axis (O1) and the second optical axis (O2) have a mutual angle α12 selected from the range of 15-90°.

9. The light generating system (1000) according to any one of the preceding claims 1-7, wherein the first light generating device (110) has a first optical axis (O1), wherein the second light generating device (120) has a second optical axis (O2), wherein the first optical axis (O1) and the second optical axis (O2) have a mutual angle α12 selected from the range of 0-15°.

10. The light generating system (1000) according to any one of the preceding claims, comprising n1 first light generating devices (110) and n2 second light generating devices (120), wherein n2<n1; and wherein the first light generating devices (110) and the second light generating devices (120) comprise solid-state light sources.

11. The light generating system (1000) according to claim 9-10, wherein n1 first light generating devices (110) and n2 second light generating devices (120) are configured in an array, wherein three or more first light generating devices (110) enclose one or more second light generating devices (120); and wherein n2/n1≤1/3.

12. The light generating system (1000) according to any one of the preceding claims, wherein the first light generating device (110) is configured to generate white first device light (111) having a correlated color temperature in a range from 2000 to 8000 K and a color rendering index of at least 80, and wherein the second light generating device (120) is configured second device light (121) having one or more second wavelengths in the UV-B and/or UV-C wavelength ranges.

13. The light generating system (1000) according to claim 12, further comprising a control system (300) and a sensor (310), wherein the control system (300) is configured to control the first light generating device (110) and the second light generating device (120) in dependence of a sensor signal of the sensor (310).

14. A lighting device (1300) selected from the group of a lamp (1), a luminaire (2), and a disinfection device, comprising the light generating system (1000) according to any one of the preceding claims.

15. A method for one or more of (i) treating air or a surface in a space, and (ii) providing light in the space; wherein:
- the space is external from the light generating system (1000) according to any one of the preceding claims 1-13 or the lighting device (1300) according to claim 14; and
- the method comprises providing system light (1001) in the space, wherein the light is generated by light generating system or by the lighting device.

## Patentansprüche

1. Lichterzeugungssystem (1000), umfassend eine erste Lichterzeugungsvorrichtung (110), eine zweite Lichterzeugungsvorrichtung (120), einen ersten Diffusor (410), einen zweiten Diffusor (420), eine erste Lichtmischkammer (510) und eine zweite Lichtmischkammer (520); wobei:
- die erste Lichterzeugungsvorrichtung (110) konfiguriert ist, um erstes Vorrichtungslicht (111) mit einer Wellenlänge im sichtbaren Wellenlängenbereich zu erzeugen; wobei die erste Lichterzeugungsvorrichtung (110) stromaufwärts des ersten Diffusors (410) und stromaufwärts des zweiten Diffusors (420) konfiguriert ist;
- die zweite Lichterzeugungsvorrichtung (120) konfiguriert ist, um zweites Vorrichtungslicht (121) mit einer zweiten Wellenlänge im UV-Wellenlängenbereich oder dem violetten Wellenlängenbereich zu erzeugen;
wobei die zweite Lichterzeugungsvorrichtung (120) stromabwärts des ersten Diffusors (410) und stromaufwärts des zweiten Diffusors (420) konfiguriert ist;
- der erste Diffusor (410) und der zweite Diffusor (420) (i) durchlässig für das erste Vorrichtungslicht (111) und (ii) streuend für das erste Vorrichtungslicht (111) sind;
- der zweite Diffusor (420) für das zweite Vorrichtungslicht (121) durchlässig ist;
- der erste Diffusor (410) eine erste UV-Reflexion R21 für das zweite Vorrichtungslicht (121) aufweist; der zweite Diffusor (420) eine zweite UV-Reflexion R22 für das zweite Vorrichtungslicht (121) aufweist, wobei R21 größer als R22 ist, wobei 25 % ≤ (R21+R22) ≤ 75 % gilt; und
- das Lichterzeugungssystem (1000) konfiguriert ist, um Systemlicht (1001) zu erzeugen, umfassend eines oder mehrere von dem ersten Vorrichtungslicht (111) und dem zweiten Vorrichtungslicht (121);
- die erste Lichtmischkammer (510) ein erstes Kammergehäuse (511) umfasst, wobei das erste Kammergehäuse (511) den ersten Diffusor (410) und einen ersten Reflektor (450) umfasst, wobei die erste Lichterzeugungsvorrichtung (110) mindestens teilweise von dem ersten Kammergehäuse (511) umschlossen ist;
- die zweite Lichtmischkammer (520) ein zweites Kammergehäuse (521) umfasst, wobei das zweite Kammergehäuse (521) den zweiten Diffusor (420) und einen zweiten Reflektor (460) umfasst, wobei die zweite Lichterzeugungsvorrichtung (120) mindestens teilweise von dem zweiten Kammergehäuse (521) umschlossen ist;
- wobei die zweite Lichtmischkammer (520) stromabwärts der ersten Lichtmischkammer (510) angeordnet ist.

2. Lichterzeugungssystem (1000) nach Anspruch 1, wobei (R21-R22) ≥ 10 % gilt.

3. Lichterzeugungssystem (1000) nach einem der vorstehenden Ansprüche, wobei 30 % ≤ (R21+R22) ≤ 70 % gilt.

4. Lichterzeugungssystem (1000) nach einem der vorstehenden Ansprüche, wobei 20 % ≤ R21 ≤ 60 % und 10 % ≤ R22 ≤ 20 % gilt.

5. Lichterzeugungssystem (1000) nach einem der vorstehenden Ansprüche, wobei der UV-Wellenlängenbereich im Wellenlängenbereich von 100 bis 380 nm liegt.

6. Lichterzeugungssystem (1000) nach einem der vorstehenden Ansprüche, wobei die erste Lichtmischkammer (510) und die zweite Lichtmischkammer (520) gestapelt sind.

7. Lichterzeugungssystem (1000) nach Anspruch 5, wobei die zweite Lichtmischkammer (520) die erste Lichtmischkammer (510) mindestens teilweise umschließt.

8. Lichterzeugungssystem (1000) nach einem der vorstehenden Ansprüche 1 bis 7, wobei die erste Lichterzeugungsvorrichtung (110) eine erste optische Achse (O1) aufweist, wobei die zweite Lichterzeugungsvorrichtung (120) eine zweite optische Achse (O2) aufweist; wobei die erste optische Achse (O1) und die zweite optische Achse (O2) einen gemeinsamen Winkel α12 aufweisen, der aus dem Bereich von 15 bis 90° ausgewählt ist.

9. Lichterzeugungssystem (1000) nach einem der vorstehenden Ansprüche 1 bis 7, wobei die erste Lichterzeugungsvorrichtung (110) eine erste optische Achse (O1) aufweist, wobei die zweite Lichterzeugungsvorrichtung (120) eine zweite optische Achse (O2) aufweist; wobei die erste optische Achse (O1) und die zweite optische Achse (O2) einen gemeinsamen Winkel α12 aufweisen, der aus dem Bereich von 0 bis 15° ausgewählt ist.

10. Lichterzeugungssystem (1000) nach einem der vorstehenden Ansprüche, umfassend n1 erste Lichterzeugungsvorrichtungen (110) und n2 zweite Lichterzeugungsvorrichtungen (120), wobei n2<n1; und wobei die ersten Lichterzeugungsvorrichtungen (110) und die zweiten Lichterzeugungsvorrichtungen (120) Festkörperlichtquellen umfassen.

11. Lichterzeugungssystem (1000) nach Anspruch 9 bis 10, wobei n1 erste Lichterzeugungsvorrichtungen (110) und n2 zweite Lichterzeugungsvorrichtungen (120) in einem Array konfiguriert sind, wobei drei oder mehr erste Lichterzeugungsvorrichtungen (110) eine oder mehrere zweite Lichterzeugungsvorrichtungen (120) umschließen; und wobei n2/n1 ≤ 1/3.

12. Lichterzeugungssystem (1000) nach einem der vorstehenden Ansprüche, wobei die erste Lichterzeugungsvorrichtung (110) konfiguriert ist, um weißes erstes Vorrichtungslicht (111) zu erzeugen, das eine korrelierte Farbtemperatur in einem Bereich von 2000 bis 8000 K und einen Farbwiedergabeindex von mindestens 80 aufweist, und wobei die zweite Lichterzeugungsvorrichtung (120) konfiguriert ist, um zweites Vorrichtungslicht (121) zu erzeugen, das eine oder mehrere zweite Wellenlängen in den UV-B- und/oder UV-C-Wellenlängenbereichen aufweist.

13. Lichterzeugungssystem (1000) nach Anspruch 12, ferner umfassend ein Steuersystem (300) und einen Sensor (310), wobei das Steuersystem (300) konfiguriert ist, um die erste Lichterzeugungsvorrichtung (110) und die zweite Lichterzeugungsvorrichtung (120) in Abhängigkeit von einem Sensorsignal des Sensors (310) zu steuern.

14. Beleuchtungsvorrichtung (1300), die aus der Gruppe einer Lampe (1), einer Leuchte (2) und einer Desinfektionsvorrichtung, umfassend das Lichterzeugungssystem (1000) nach einem der vorstehenden Ansprüche, ausgewählt ist.

15. Verfahren für eines oder mehrere von (i) Behandeln von Luft oder einer Oberfläche in einem Raum, und (ii) Bereitstellen von Licht in dem Raum; wobei:
- der Raum außerhalb des Lichterzeugungssystems (1000) nach einem der vorstehenden Ansprüche 1 bis 13 oder der Beleuchtungsvorrichtung (1300) nach Anspruch 14 ist; und
- das Verfahren das Bereitstellen von Systemlicht (1001) in dem Raum umfasst, wobei das Licht durch ein Lichterzeugungssystem oder durch die Beleuchtungsvorrichtung erzeugt wird.

## Revendications

1. Système de génération de lumière (1000) comprenant un premier dispositif de génération de lumière (110), un second dispositif de génération de lumière (120), un premier diffuseur (410), un second diffuseur (420), une première chambre de mélange de lumière (510) et une seconde chambre de mélange de lumière (520) ; dans lequel :
- le premier dispositif de génération de lumière (110) est configuré pour générer une première lumière de dispositif (111) ayant une première longueur d'onde dans la plage des longueurs d'onde visibles ; dans lequel le premier dispositif de génération de lumière (110) est configuré en amont du premier diffuseur (410) et en amont du second diffuseur (420) ;
- le second dispositif de génération de lumière (120) est configuré pour générer une seconde lumière de dispositif (121) ayant une seconde longueur d'onde dans la plage des longueurs d'onde UV ou dans la plage des longueurs d'onde violettes ; dans lequel le second dispositif de génération de lumière (120) est configuré en aval du premier diffuseur (410) et en amont du second diffuseur (420) ;
- le premier diffuseur (410) et le second diffuseur (420) sont (i) transmissifs pour la première lumière de dispositif (111) et (ii) diffusants pour la première lumière de dispositif (111) ;
- le second diffuseur (420) est transmissif pour la seconde lumière de dispositif (121) ;
- le premier diffuseur (410) a une première réflexion UV R21 pour la seconde lumière de dispositif (121) ; le second diffuseur (420) a une seconde réflexion UV R22 pour la seconde lumière de dispositif (121), dans lequel R21 est supérieur à R22, dans lequel 25 % ≤ (R21 + R22) ≤ 75 % s'applique ; et
- le système de génération de lumière (1000) est configuré pour générer une lumière de système (1001) comprenant l'une ou plusieurs parmi la première lumière de dispositif (111) et la seconde lumière de dispositif (121) ;
- la première chambre de mélange de lumière (510) comprend une première enceinte de chambre (511), dans lequel la première enceinte de chambre (511) comprend le premier diffuseur (410) et un premier réflecteur (450), dans lequel le premier dispositif de génération de lumière (110) est au moins partiellement enfermé par la première enceinte de chambre (511) ;
- la seconde chambre de mélange de lumière (520) comprend une seconde enceinte de chambre (521), dans lequel la seconde enceinte de chambre (521) comprend le second diffuseur (420) et un second réflecteur (460), dans lequel le second dispositif de génération de lumière (120) est au moins partiellement enfermé par la seconde enceinte de chambre (521) ;
- dans lequel la seconde chambre de mélange de lumière (520) est configurée en aval de la première chambre de mélange de lumière (510).

2. Système de génération de lumière (1000) selon la revendication 1, dans lequel (R21-R22) ≥ 10 % s'applique.

3. Système de génération de lumière (1000) selon l'une quelconque des revendications précédentes, dans lequel 30 % ≤ (R21 + R22) ≤ 70 % s'applique.

4. Système de génération de lumière (1000) selon l'une quelconque des revendications précédentes, dans lequel 20 % ≤ R21 ≤ 60 % et 10 % ≤ R22 ≤ 20 % s'applique.

5. Système de génération de lumière (1000) selon l'une quelconque des revendications précédentes, dans lequel la plage des longueurs d'onde UV est la plage de longueurs d'onde allant de 100 à 380 nm.

6. Système de génération de lumière (1000) selon l'une quelconque des revendications précédentes, dans lequel la première chambre de mélange de lumière (510) et la seconde chambre de mélange de lumière (520) sont empilées.

7. Système de génération de lumière (1000) selon la revendication 5, dans lequel la seconde chambre de mélange de lumière (520) enferme au moins partiellement la première chambre de mélange de lumière (510).

8. Système de génération de lumière (1000) selon l'une quelconque des revendications précédentes 1 à 7, dans lequel le premier dispositif de génération de lumière (110) a un premier axe optique (O1), dans lequel le second dispositif de génération de lumière (120) a un second axe optique (O2), dans lequel le premier axe optique (O1) et le second axe optique (O2) ont un angle mutuel α12 choisi dans la plage allant de 15 à 90 °.

9. Système de génération de lumière (1000) selon l'une quelconque des revendications précédentes 1 à 7, dans lequel le premier dispositif de génération de lumière (110) a un premier axe optique (O1), dans lequel le second dispositif de génération de lumière (120) a un second axe optique (O2), dans lequel le premier axe optique (O1) et le second axe optique (O2) ont un angle mutuel α12 choisi dans la plage allant de 0 à 15°.

10. Système de génération de lumière (1000) selon l'une quelconque des revendications précédentes, comprenant n1 premiers dispositifs de génération de lumière (110) et n2 seconds dispositifs de génération de lumière (120), dans lequel n2 < n1 ; et dans lequel les premiers dispositifs de génération de lumière (110) et les seconds dispositifs de génération de lumière (120) comprennent des sources de lumière à l'état solide.

11. Système de génération de lumière (1000) selon la revendication 9 ou 10, dans lequel n1 premiers dispositifs de génération de lumière (110) et n2 seconds dispositifs de génération de lumière (120) sont configurés selon un réseau, dans lequel trois ou plusieurs premiers dispositifs de génération de lumière (110) enferment un ou plusieurs seconds dispositifs de génération de lumière (120) ; et dans lequel n2/n1≤1/3.

12. Système de génération de lumière (1000) selon l'une quelconque des revendications précédentes, dans lequel le premier dispositif de génération de lumière (110) est configuré pour générer une première lumière de dispositif blanche (111) ayant une température de couleur corrélée dans une plage allant de 2000 à 8000 K et un indice de rendu des couleurs d'au moins 80, et dans lequel le second dispositif de génération de lumière (120) est configuré pour une seconde lumière de dispositif (121) ayant une ou plusieurs secondes longueurs d'onde dans les plages de longueurs d'onde UV-B et/ou UV-C.

13. Système de génération de lumière (1000) selon la revendication 12, comprenant en outre un système de commande (300) et un capteur (310), dans lequel le système de commande (300) est configuré pour commander le premier dispositif de génération de lumière (110) et le second dispositif de génération de lumière (120) en fonction d'un signal de capteur du capteur (310).

14. Dispositif d'éclairage (1300) choisi dans le groupe d'une lampe (1), un luminaire (2) et un dispositif de désinfection, comprenant le système de génération de lumière (1000) selon l'une quelconque des revendications précédentes.

15. Procédé destiné à un ou plusieurs parmi (i) le traitement de l'air ou d'une surface dans un espace, et (ii) la fourniture de lumière dans l'espace ; dans lequel :
- l'espace est externe au système de génération de lumière (1000) selon l'une quelconque des revendications précédentes 1 à 13 ou au dispositif d'éclairage (1300) selon la revendication 14 ; et
- le procédé comprend la fourniture d'une lumière de système (1001) dans l'espace, dans lequel la lumière est générée par le système de génération de lumière ou par le dispositif d'éclairage.
